# EUROPÄISCHE PATENTSCHRIFT

(11) **EP 1 545 553 B1**
(45) Veröffentlichungstag und Bekanntmachung des Hinweises auf die Patenterteilung: **13.07.2011**
(21) Anmeldenummer: 03766336.6
(22) Anmeldetag: 29.07.2003
(51) Int. Cl.: A61K 31/685, A61P 35/00

(54) **ANWENDUNG VON ALKYLPHOSPHOCHOLINEN IN KOMBINATION MIT ANTITUMORMEDIKAMENTEN**
USE OF ALKYL PHOSPHOCHOLINES IN COMBINATION WITH ANTITUMOR MEDICAMENTS
UTILISATION D'ALKYLPHOSPHOCHOLINES EN ASSOCIATION AVEC DES MEDICAMENTS ANTITUMORAUX

(30) Priorität: 30.07.2002 US 399615 P
(43) Veröffentlichungstag der Anmeldung: 29.06.2005
(60) Teilanmeldung: 10010451.2
(73) Patentinhaber: Æterna Zentaris GmbH, 60314 Frankfurt am Main (DE)
(72) Erfinder: ENGEL, Jürgen, 63755 Alzenau (DE); GÜNTHER, Eckhard, 63477 Maintal (DE); SINDERMANN, Herbert, 63110 Rodgau (DE)
(74) Vertreter: Polypatent
(86) Internationale Anmeldenummer: PCT/EP2003/008346
(87) Internationale Veröffentlichungsnummer: WO 2004/012744

(56) Entgegenhaltungen:
- WO-A-00/33917
- WO-A-02/066019
- WO-A-03/055522
- HILGARD P ET AL: "Heterocyclic alkylphospholipids with an improved therapeutic range." ADVANCES IN EXPERIMENTAL MEDICINE AND BIOLOGY. UNITED STATES 1996, Bd. 416, 1996, Seiten 157-164, XP002256712 ISSN: 0065-2598
- STEKAR J ET AL: "Opposite effect of miltefosine on the antineoplastic activity and haematological toxicity of cyclophosphamide." EUROPEAN JOURNAL OF CANCER (OXFORD, ENGLAND: 1990) ENGLAND 1995, Bd. 31A, Nr. 3, 1995, Seiten 372-374, XP002256713 ISSN: 0959-8049
- SPRUSS T ET AL: "ANTITUMOUR ACTIVITY OF MILTEFOSINE ALONE AND AFTER COMBINATION WITHPLATINUM COMPLEXES ON MXT MOUSE MAMMARY CARCINOMA MODELS" JOURNAL OF CANCER RESEARCH AND CLINICAL ONCOLOGY, SPRINGER INTERNATIONAL, BERLIN, DE, Bd. 119, Nr. 3, 1993, Seiten 142-149, XP000905599 ISSN: 0171-5216
- GEORGIEVA MILKA C ET AL: "Combination effects of alkylphosphocholines and gemcitabine in malignant and normal hematopoietic cells." CANCER LETTERS. IRELAND 28 AUG 2002, Bd. 182, Nr. 2, 28. August 2002 (2002-08-28), Seiten 163-174, XP002256714 ISSN: 0304-3835
- BERKOVIC D ET AL: "The influence of 1-beta-D-arabinofuranosylcytosine on the metabolism of phosphatidylcholine in human leukemic HL 60 and Raji cells." LEUKEMIA: OFFICIAL JOURNAL OF THE LEUKEMIA SOCIETY OF AMERICA, LEUKEMIA RESEARCH FUND, U.K. ENGLAND DEC 1997, Bd. 11, Nr. 12, Dezember 1997 (1997-12), Seiten 2079-2086, XP002256715 ISSN: 0887-6924
- DATABASE MEDLINE [Online] US NATIONAL LIBRARY OF MEDICINE (NLM), BETHESDA, MD, US; Februar 1991 (1991-02) SHOJI M ET AL: "Effects of hexadecylphosphocholine on protein kinase C and TPA-induced differentiation of HL60 cells." Database accession no. NLM2051897 XP002256716 & LIPIDS. UNITED STATES FEB 1991, Bd. 26, Nr. 2, Februar 1991 (1991-02), Seiten 145-149, ISSN: 0024-4201
- HILGARD P. ET AL: 'ALKYLPHOSPHOCHOLINES: A NEW CLASS OF MEMBRANE-ACTIVE ANTICANCER AGENTS' CANCER CHEMOTHERAPY AND PHARMACOLOGY, SPRINGER VERLAG, BERLIN Bd. 32, Nr. 2, 01 Januar 1993, Seiten 90 - 95, XP008014958 ISSN: 0344-5704

## Beschreibung

Alkylphosphocholine sind eine neue Klasse organischer Verbindungen, die vielfältige antineoplastische Aktivitäten zeigen (M. Lohmeyer und R. Bittman; Antitumor ether lipids and alkylphosphocholines, DOF, 19 (11), 1021-1037 (1994)). Die Wirkung der Alkylphosphocholine kann dabei auf verschiedenen molekularen und biochemischen Mechanismen beruhen, die zum Teil auf Ebene der Plasmamembran der Zelle stattfinden. Gut bekannt ist der Einfluß der Alkylphosphocholine auf den Inositol-Metabolismus, die Interaktion mit Phospholipasen oder die Hemmung der Protein Kinase C und damit eine generelle Beeinflussung der Signaltransduktion der Zelle durch diese Stoffklasse (K. Maly, F. Überall, C. Schubert, E. Kindler, J. Stekar, H. Brachwitz und H. H. Grunicke, Interference of new alkylphospholipid analogues with mitogenic signal transduction, Anti-Cancer Drug Design, 10, 411-425 (1995)). So zeigt das Alkylphosphocholine Perifosine wachstums-inhibitorische Eigenschaften gegenüber verschiedenen Melanom-, CNS-, Lunge-, Colon-, Prostata- und Brustkrebszelllinien mit einer IC₅₀ im Bereich von 0.2 - 20 µM (P. Hilgard, T. Klenner, J. Stekar, G. Nössner, B. Kutscher und J. Engel; D-21266, a New Heterocyclic Alkylphospholipid with Antitumor Activity, Eur. J. Cancer, 33 (3), 442-446 (1997)). Des weiteren ist bekannt, daß Perifosine Tumorzellen in der G₁-S und G₂-M Phase des Zellzyklus blockiert (V. Patel, T. Lahusen, T. Sy, E. A. Sausville, J. S. Gutkind und A. M. Senderowicz; Perifosine, a Novel Alkylphospholipid, Induces p21waf1 Expression in Squamous Carcinoma Cells through a p53-independent Pathway, Leading to Loss in Cyclin-dependent Kinase Activity and Cell Cycle Arrest, Cancer Research 62, 1401-1409 (2002)).

Es ist bekannt, daß der Einsatz von Alkylphosphocholinen vor oder zusammen mit der Strahlentherapie zu synergistischen Effekten bei der Behandlung von Tumoren führt (G.A. Ruitter, M. Verheijl, S. F. Zerp und W. J. van Blitterswijk; Alkyl-Lysophospholipids as Anticancer Agents and Enhancers of Radiation-Induced Apoptosis, Int. J. Radiation Oncology Biol. Phys., 49 (2), 415-420, 2001). Es wurde ferner berichtet, daß verschiedene Glycero-3-phospholipide, z.B. ET-18-OCH₃ in Kombination mit verschiedenen DNA-interagierenden Substanzen oder Tubulin-Bindern die Antitumor Aktivität in-vitro an verschiedenen Tumorzelllinien erhöhen (A. Noseda, M. E. Berens, J. G. White und E. J. Modest; In vitro antiproliferative activity of combinations of ether lipid analogs and DNA-Interactive agents against human tumor cells, Cancer Res., 48 (7), 1788-1791 (1988); P. Principe, H. Coulomb, C. Broquet und P. Braquet; Evaluation of combinations of antineoplastic ether phospholipids and chemotherapeutic drugs, Ant-Cancer Drugs, 3 (6), 577-587 (1992); P. Principe, H. Coulomb, J.-M. Mencia-Huerta, C. Broquet und P. Braquet; Synergistic cytotoxic effect of aza-alkylphospholipids in association with chemotherapeutic drugs, J. Lipid Mediators Cell Signalling, 10 (1-2), 171-173 (1994)).

Es konnte jetzt überraschend gezeigt werden, daß lineare Alkylphosphocholine der allgemeinen Formel II geeignet sind, in einer erfindungsgemäßen Kombination mit anderen Arzneimitteln zur Behandlung von gut- und bösartigen Tumorerkrankungen am Menschen und Säugetier eingesetzt zu werden. Dabei können die Verbindungen der allgemeinen Formel II in einer erfindungsgemäßen Kombination mit Antitumor-Substanzen eingesetzt werden. Bei den Antitumor-Substanzen handelt es sich um. Antimetabolite. Diese Antitumor-Substanzen können ausgewählt aber nicht darauf beschränkt sein aus: Methotrexat, 5-Fluorouracil, Fludarabin, Gemcitabin und Cytarabin.

Die der Erfindung zugrunde liegenden Alkylphosphocholine der allgemeinen Formel II können in Form von Fertigarzneimitteln zur Anwendung gelangen.

Die der Erfindung zugrunde liegenden Verbindungen werden durch die allgemeine Formel II beschrieben: 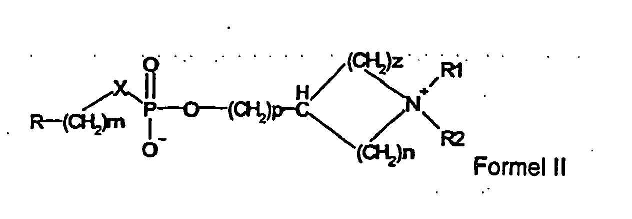 wobei unabhängig voneinander
- n, m, p, z: eine ganze Zahl zwischen 0 und 4 bedeuten;
- X: O, S, NH;
- R: H, einen geradkettigen oder verzweigten (C₁-C₂₀)-Alkyl-Rest, welcher gesättigt oder mit ein bis drei Doppel- und/oder Dreifachbindungen ungesättigt sein kann und welcher unsubstituiert oder wahlweise an dem gleichen oder an verschiedenen C-Atomen mit ein, zwei oder mehreren Halogen, Nitro, Cyano, Hydroxy, (C₁-C₆)-Alkoxy, Amino, Mono-(C₁-C₄) Alkylamino oder Di- (C₁-C₄)-Alkylamino Resten substituiert sein kann, bedeutet.
- R₁, R₂: unabhängig von einander H, einen geradkettigen oder verzweigten (C₁₋ C₆)-Alkyl-Rest, vorzugsweise Methyl und Ethyl, einen (C₃-C₇)-Cycloalky- Rest und welcher unsubstituiert oder wahlweise an dem gleichen oder an verschiedenen C-Atomen mit ein, zwei oder mehreren Halogen, Nitro, Cyano, Hydroxy, (C₁-C₆)-Alkoxy, Amino, Mono-(C₁-C₄) Alkylamino oder Di- (C₁-C₄)-Alkylamino Resten substituiert sein kann, bedeutet.

Gemäß der Erfindung wird mindestens eine der Erfindung zugrunde liegenden Verbindungen gemäß der allgemeinen Formel II zur Herstellung einer Anneimittels zur Bekämpfung von Tumoren beim Menschen und in Säugetieren verwendet, welche dadurch gekennzeichnet ist, daß sie dem Menschen oder einem Säugetier in einer für die Tumorbehandlung wirksamen Menge vor oder während einer Behandlung mit zugelassenen Antitumor-Substanzen verabreicht wird.

Die für die Behandlung zu verabreichende therapeutisch effektive Dosis der jeweiligen der Erfindung zugrunde liegenden Verbindung der allgemeinen Formel II richtet sich u.a. nach der Art und dem Stadium der Tumorerkrankung, dem Alter und Geschlecht des Patienten, der Art der Verabreichung und der Dauer der Behandlung.

Die der Erfindung zugrunde liegenden Verbindungen können in einem Arzneimittel als flüssige, halbfeste und feste Arzneiformen verabreicht werden. Dies erfolgt in der jeweils geeigneten Weise in Form von Aerosolen, Pulver, Puder und Streupuder, Tabletten, Dragees, Emulsionen, Schäume, Lösungen, Suspensionen, Gele, Salben, Pasten, Pillen, Pastillen, Kapseln oder Suppositorien.

### Referenzbeispiele:

### 1. Gabe von Perifosine (D-21 266) in Kombination mit Cisplatin

- In-vivo Versuch:: DMBA induziertes Mamma-Karzinom Modell an der Ratte
- Versuchstier:: Sprague-Dawley Ratte, weiblich
- Durchführung:: Die Induktion des Mamma-Karzinoms erfolgte durch einmalige orale Gabe von DMBA. Die Tiere erhielten von Tag 0 bis Tag 14 Perifosin und wurden bis Tag 42 beobachtet. Das Gewicht der Tumormasse wurde mittels Palpation und Vergleich mit Plastikmodellen ermittelt. Das Ausgangsgewicht wird gleich 100% gesetzt.
- Gabe:: Perifosin 14 x 6.81 mg/kg p.o. Cisplatin 4 x 1 mg/kg i.p.
- Effekt:: Die Reduktion des Tumors war durch die Kombinationsbehandlung deutlich stärker und länger als durch die jeweilige Einzelbehandlung.

| Behandlung | Tumor Ausgangsgewicht [g] | Tag 21 Veränderung in [%] | p Test vs. Kontrolle |
|---|---|---|---|
| Kontrolle | 1.0 | 875 | - |
| Perifosin (D-21266) | 0.9 | -25 | <0.001 |
| Cisplatin | 0.9 | 410 | 0.120 |
| Perifosin (D-21266) + Cisplatin | 0.8 | -75 | <0.001 |

### 2. Gabe von Perifosine in Kombination mit Cyclophosphamid

- In-vivo Versuch:: DMBA induziertes Mamma-Karzinom Modell an der Ratte
- Versuchstier:: Sprague-Dawley Ratte, weiblich
- Durchführung:: Die Induktion des Mamma-Karzinoms erfolgte durch einmalige orale Gabe von DMBA. Die Tiere erhielten von Tag 0 bis Tag 14 Perifosin und wurden bis Tag 42 beobachtet. Das Gewicht der Tumormasse wurde mittels Palpation und Vergleich mit Plastikmodellen ermittelt. Das Ausgangsgewicht wird gleich 100% gesetzt.
- Gabe:: Perifosin 14 x 6.81 mg/kg p.o. Cyclophosphamid 100 mg/kg, VZ 0, i.v.
- Effekt:: Die Reduktion des Tumors war durch die Kombinationsbehandlung deutlich stärker und länger als durch die jeweilige Einzelbehandlung.

| Behandlung | Tumor Ausgangsgewicht [g] | Tag 21 Veränderung in [%] | p Test vs. Kontrolle |
|---|---|---|---|
| Kontrolle | 1.0 | 875 | - |
| Perifosin (D-21266) | 0.9 | -25 | <0.001 |
| Cyclophosphamid | 0.9 | 500 | 0.011 |
| Perifosin (D-21266) + Cyclophosphamid | 0.8 | -83.3 | <0.001 |

### 3. Gabe von Perifosine in Kombination mit Adriamycin

- In-vivo Versuch:: DMBA induziertes Mamma-Karzinom Modell an der Ratte
- Versuchstier:: Sprague-Dawley Ratte, weiblich
- Durchführung:: Die Induktion des Mamma-Karzinoms erfolgte durch einmalige orale Gabe von DMBA. Die Tiere erhielten von Tag 0 bis Tag 14 Perifosin und wurden bis Tag 42 beobachtet. Das Gewicht der Tumormasse wurde mittels Palpation und Vergleich mit Plastikmodellen ermittelt. Das Ausgangsgewicht wird gleich 100% gesetzt.
- Gabe:: Perifosin 14 x 6.81 mg/kg p.o. Adriamycin 4 x 2.15 mg/kg i.p.
- Effekt:: Die Reduktion des Tumors war durch die Kombinationsbehandlung deutlich stärker und länger als durch die jeweilige Einzelbehandlung.

| Behandlung | Tumor Ausgangsgewicht [g] | Tag 21 Veränderung in [%] | p Test vs. Kontrolle |
|---|---|---|---|
| Kontrolle | 1.0 | 875 | - |
| Perifosin (D-21266) | 0.9 | -25 | <0.001 |
| Adriamycin | 1.0 | 781.3 | 0.197 |
| Perifosin (D-21266) + Adriamycin | 01.0 | -70 | <0.001 |

## Patentansprüche

1. Verwendung von Alkylphosphocholinen der allgemeinen Formel II 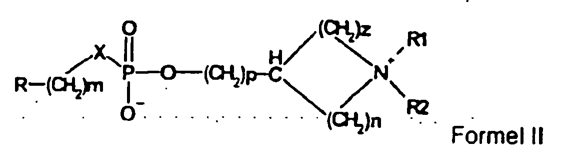 wobei unabhängig voneinander:
n, m, p, z eine ganze Zahl zwischen 0 und 4 bedeuten;
X O, S, NH bedeuten;
R H, einen geradkettigen oder verzweigten (C₁-C₂₀)-Alkyl-Rest, welcher gesättigt oder mit ein bis drei Doppel- und/oder Dreifachbindungen ungesättigt sein kann und welcher unsubstituiert oder wahlweise an dem gleichen oder an verschiedenen C-Atomen mit ein, zwei oder mehreren Halogen, Nitro, Cyano, Hydroxy, (C₁-C₆)-Alkoxy. Amino, Mono-(C₁-C₄) Alkylamino oder Di- (C₁-C₄)-Alkylamino Resten substituiert sein kann, bedeutet;
R₁, R₂ unabhängig von einander H, einen geradkettigen oder verzweigten (C₁- C₆)-Alkyl-Rest, vorzugsweise Methyl und Ethyl, einen (C₃-C₇)- Cycloalky-Rest und welcher unsubstituiert oder wahlweise an dem gleichen oder an verschiedenen C-Atomen mit ein, zwei oder mehreren Halogen, Nitro, Cyano, Hydroxy, (C₁-C₆)-Alkoxy, Amino, Mono-(C₁-C₄) Alkylamino oder Di- (C₁-C₄)-Alkylamino Resten substituiert sein kann, bedeutet,
zur Herstellung eines Arzneimittels zur Behandlung von gutartigen und bösartigen Tumorerkrankungen vor und/oder während der Behandlung mit einem zugelassenen Antitumormedikament, das ein Antimetabolit ist.

2. Verwendung nach Anspruch 1, wobei unabhängig voneinander:
m, p die ganze Zahl 1 bedeutet;
n, z die ganze Zahl 2 bedeutet;
X O bedeutet;
R H, einen geradkettigen oder verzweigten (C₁-C₁₇)-Alkyl-Rest, welcher gesättigt oder mit ein bis drei Doppel- und/oder Dreifachbindungen ungesättig sein kann, bedeutet;
R₁, R₂ unabhängig voneinander H, einen geradkettigen oder verzweigten (C₁-C₆)- Alkyl-Rest, vorzugsweise Methyl und Ethyl, einen (C₃-C₇)-Cycloalkyl-Rest sein kann, bedeutet.

3. Verwendung nach Anspruch 1, wobei das Alkylphosphocholin Octadecyl-(1,1-dimethyl-piperidino-4-yl)-phosphat ist.

4. Verwendung nach den Ansprüchen 1 bis 3, wobei das Alkylphosphocholin in einer für die Behandlung wirksamen therapeutischen Dosis vor und/oder während der Behandlung mit demzugelassenen Antitumormedikament verwendet wird.

5. Verwendung nach den Ansprüchen 1 bis 4, **dadurch gekennzeichnet, dass** das Arzneimittel neben dem Alkylphosphocholin die üblichen pharmazeutischen Träger, Hilfsstoffe und/oder Verdünnungsmittel enthält.

## Claims

1. Use of alkylphosphocholines of general formula II 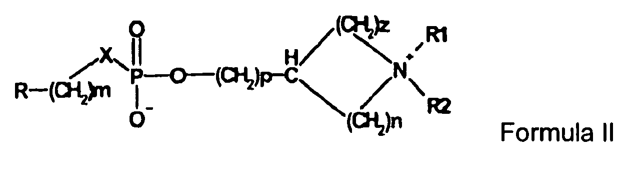 wherein
n, m, p, z independently represent an integer between 0 and 4;
X represents O, S, NH;
R represents H, a linear or branched C₁-C₂₀ alkyl radical which may be saturated or unsaturated with one to three double and/or triple bonds, and which may be unsubstituted or optionally substituted on the same or different C atoms with one, two or more of halogen, nitro, cyano, hydroxy, C₁-C₆ alkoxy, amino, mono(C₁-C₄ alkyl)amino or di(C₁-C₄ alkyl)amino radicals;
R₁, R₂ each independently represent H, a linear or branched C₁-C₆ alkyl radical, preferably methyl and ethyl, a C₃-C₇ cycloalkyl radical which may be unsubstituted or optionally substituted on the same or different C atoms with one, two or more of halogen, nitro, cyano, hydroxy, C₁-C₆ alkoxy, amino, mono(C₁-C₄ alkyl)- amino or di(C₁-C₄ alkyl)amino radicals;
in the preparation of a medicament for the treatment of benign and malignant tumor diseases prior to and/or during treatment with an approved antitumor medicament which is an antimetabolite.

2. The use according to claim 1, wherein
m, p independently represent the integer 1;
n, z independently represent the integer 2;
X represents O;
R represents H, a linear or branched C₁-C₁₇ alkyl radical which may be saturated or unsaturated with one to three double and/or triple bonds;
R₁, R₂ each independently represent H, a linear or branched C₁-C₆ alkyl radical, preferably methyl and ethyl, a C₃-C₇ cycloalkyl radical.

3. The use according to claim 1, wherein said alkylphosphocholine is octadecyl 1,1-dimethylpiperidino-4-yl phosphate.

4. The use according to claims 1 to 3, wherein said alkylphosphocholine is used in a therapeutic dose effective for treatment prior to and/or during treatment with said approved antitumor medicament.

5. The use according to claims 1 to 4, **characterized in that** the drug includes common pharmaceutical vehicles, adjuvants and/or diluents in addition to the alkylphosphocholine.

## Revendications

1. Utilisation d'alkylphosphocholines de formule générale II 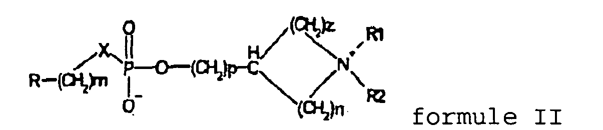 dans laquelle, indépendamment les uns des autres :
n, m, p, z représentent un nombre entier entre 0 et 4 ;
X représente O, S, NH ;
R représente un atome de H, un radical alkyle (en C₁-C₂₀) linéaire ou ramifié, qui peut être saturé ou insaturé avec une à trois doubles et/ou triples liaisons et qui peut être non substitué ou optionnellement substitué sur le même atome de C ou sur différents atomes de C avec un, deux ou plusieurs atomes d'halogène, radicaux nitro, cyano, hydroxy, alcoxy (en C₁-C₆), amino, mono(alkyle en C₁-C₄)amino ou di(alkyle en C₁-C₄)amino ;
R₁, R₂ représentent, indépendamment l'un de l'autre, un atome de H, un radical alkyle (en C₁-C₆) linéaire ou ramifié, de préférence méthyle et éthyle, un radical cycloalkyle (en C₃-C₇) et qui peut être non substitué ou optionnellement substitué sur le même atome de C ou sur différents atomes de C avec un, deux ou plusieurs atomes d'halogène, radicaux nitro, cyano, hydroxy, alcoxy (en C₁-C₆), amino, mono(alkyle en C₁-C₄)amino ou di(alkyle en C₁-C₄)amino ;
pour la préparation d'un médicament destiné au traitement des tumeurs bénignes et malignes avant et/ou pendant le traitement avec un médicament anti-tumoral autorisé, qui est un antimétabolite.

2. Utilisation selon la revendication 1, dans laquelle, indépendamment les uns des autres :
m, p représentent le nombre entier 1 ;
n, z représentent le nombre entier 2 ;
X représente O ;
R représente un atome de H, un radical alkyle (en C₁-C₁₇) linéaire ou ramifié, qui peut être saturé ou insaturé avec une à trois doubles et/ou triples liaisons ;
R₁, R₂ représentent, indépendamment l'un de l'autre, un atome de H, un radical alkyle (en C₁-C₆) linéaire ou ramifié, de préférence méthyle et éthyle, un radical cycloalkyle (en C₃-C₇).

3. Utilisation selon la revendication 1, dans laquelle l'alkylphosphocholine est l'octadécyl-(1,1-diméthylpipéridino-4-yl)-phosphate.

4. Utilisation selon les revendications 1 à 3, dans laquelle l'alkylphosphocholine est utilisée en une dose thérapeutique efficace pour le traitement avant et/ou pendant ) le traitement avec le médicament anti-tumoral autorisé.

5. Utilisation selon les revendications 1 à 4, **caractérisée en ce que** le médicament comprend, en plus de l'alkylphosphocholine, les véhicules, auxiliaires et/ou diluants pharmaceutiques usuels.
